# FASCICULE DE BREVET EUROPEEN

(11) **EP 1 201 659 B1**
(45) Date de publication et mention de la délivrance du brevet: **19.05.2004**
(21) Numéro de dépôt: 01402707.2
(22) Date de dépôt: 19.10.2001
(51) Int. Cl.: C07D 263/44, C07D 269/02

(54) **Procédé de préparation des N-carboxyanhydrides**
Verfahren zur Herstellung von N-Carboxyanhydriden
Process for the preparation of N-carboxyanhydrides

(30) Priorité: 30.10.2000 FR 0013906
(43) Date de publication de la demande: 02.05.2002
(73) Titulaire: ISOCHEM, 75194 Paris Cedex 04 (FR)
(72) Inventeur: Cornille, Fabrice, 91440 Bures sur Yvette (FR); Copier, Jean-Luc, 91290 Arpajon (FR); Senet, Jean-Pierre, 77760 Buthiers (FR); Robin, Yves, 91710 Vert le Petit (FR)
(74) Mandataire: Waligorski, Carol

(56) Documents cités:
- FR-A- 1 561 268
- FR-A- 2 000 198
- GB-A- 1 210 719
- US-A- 3 369 026

## Description

L'invention concerne un procédé amélioré de préparation des N-carboxyanhydrides à partir des amino-acides correspondants et du phosgène, du diphosgène ou du triphosgène.

Les N-carboxyanhydrides (abréviation NCA) obtenus à partir des α-, β- ou γ-amino-acides sont des composés très utiles en raison de l'activation de leur fonction acide. Ils permettent en effet la réaction de cette fonction acide avec toute entité nucléophile. Ainsi l'obtention de la fonction amide par réaction avec une fonction amine est facilitée. De ce fait, ils polymérisent facilement et sont utilisés pour former des peptides. La liaison ester par réaction avec un alcool se crée également aisément. Ils sont aussi intéressants lorsqu'on souhaite réduire une fonction acide.

Plusieurs procédés sont connus pour préparer les N-carboxyanhydrides. Un des plus courants et des plus directs est le procédé selon lequel on fait réagir un amino-acide ou son chlorhydrate avec du phosgène, du diphosgène ou du triphosgène, dans un milieu solvant.

Le schéma réactionnel général avec le phosgène est le suivant : 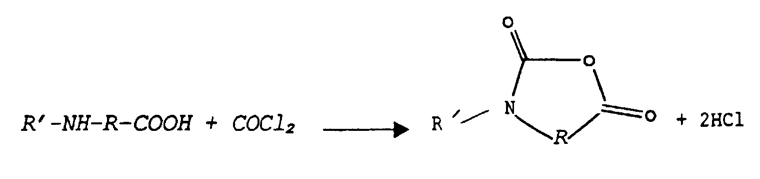 dans lequel R représente le radical principal de l'α-, β-ou γ-amino-acide et R' représente un atome d'hydrogène ou le radical du groupe amino secondaire de l'amino-acide, R' pouvant former un cycle avec R.

On constate qu'outre du N-carboxyanhydride, il se forme aussi de l'acide chlorhydrique en grande quantité, c'est-à-dire 2 moles par mole de NCA. L'acide chlorhydrique est très réactif. Sa présence dans le milieu entraîne des réactions secondaires et l'apparition de sous-produits chlorés. Ces impuretés chlorées qui restent dans les NCA produits sont tout à fait indésirables aussi bien en terme de qualité qu'en terme de rendement. En effet, elles gênent énormément la réaction de polymérisation des NCA. Pour que cette polymérisation s'effectue convenablement, il est nécessaire que la quantité de composés chlorés contenus dans les NCA monomères soit suffisamment faible. Ainsi le taux de chlore hydrolysable doit généralement être inférieur à 0,05 % en poids.

Or selon les procédés connus, lorsque la réaction est effectuée sans la présence d'un composé basique, il est difficile d'obtenir de façon répétitive un taux de chlore hydrolysable aussi faible. D'un autre côté, lorsqu'on ajoute un composé basique pour neutraliser l'acide chlorhydrique, la polymérisation des NCA, non souhaitée à ce stade, est activée et risque alors de se produire dans le milieu. Selon le procédé décrit dans le brevet GB n° 1.210.719, les composés utilisés pour neutraliser l'acide chlorhydrique sont des sels minéraux tels que des oxydes ou des cyanures d'argent, de plomb ou de mercure.

Selon le procédé décrit dans le brevet FR n° 1 561 268, la réaction est effectuée dans des hydrocarbures halogénés comme solvants.

Selon le brevet FR n° 2 000 198, une amélioration au procédé précédent consiste à utiliser un mélange de solvants constitué d'hydrocarbures aromatiques et de nitriles organiques.

Par ailleurs, une des autres difficultés des procédés antérieurs est le choix du solvant. Il a en effet été constaté que dans des solvants tels que les esters aliphatiques comme l'acétate d'éthyle ou des solvants aprotiques apolaires comme le dichlorométhane ou le toluène, la réaction de formation des NCA est généralement très lente et incomplète. Dans un solvant de la famille des éthers tel que le tétrahydrofuranne, ou le dioxanne, la réaction est plus rapide, mais ces solvants ne sont pas complètement inertes vis-à-vis du phosgène et de l'acide chlorhydrique, ce qui génère d'autres impuretés.

Il existait, par conséquent, un besoin d'améliorer le procédé existant dans lequel l'amino-acide est mis à réagir directement avec le phosgène, le diphosgène ou le triphosgène afin d'obtenir les NCA avec de meilleurs rendements et une pureté améliorée, notamment possédant un taux de chlore hydrolysable inférieur à 0,05 %. La diminution de la durée de la réaction, dans les solvants les plus inertes, était aussi fortement souhaitable.

Le procédé selon la présente invention répond à ces besoins. Selon ce procédé, on prépare les N-carboxyanhydrides par réaction de l'α-, β- ou γ-amino-acide correspondant ou d'un de ses sels, avec le phosgène, le diphosgène et/ou le triphosgène, dans un milieu solvant, en présence, pendant toute ou une partie de la durée de la réaction, d'un composé organique insaturé qui possède une ou plusieurs doubles liaisons de type éthylénique, dont le reste de la molécule est inerte vis-à-vis des composés présents dans le milieu, et dont l'un des carbones d'au moins une double liaison éthylénique est complètement substitué par des substituants différents des atomes d'halogène.

Grâce à ce nouveau procédé, les problèmes qui se posaient dans l'art antérieur sont résolus. L'acide chlorhydrique, qui se dégage, se fixe au fur et à mesure de sa formation sur la ou les doubles liaisons éthyléniques du composé insaturé. Les nombreuses réactions secondaires provoquées par l'acide chlorhydrique sont ainsi supprimées et, en conséquence, l'apparition des impuretés gênantes également. De plus, on favorise également le déplacement de l'équilibre réactionnel dans le sens de l'obtention du NCA souhaité et en conséquence la cinétique de la réaction est accélérée.

On a aussi trouvé que dans le cas de la transformation des amino-acides dont la fonction amine est secondaire, la présence de ce composé insaturé rendait inutile l'ajout, dans le milieu, d'une amine tertiaire telle que la triéthylamine ou la N-méthylmorpholine. Une telle amine était pourtant jusqu'à présent, jugée nécessaire par l'homme de métier pour effectuer la cyclisation à partir du chlorure de carbamoyle qui se forme tout d'abord dans le milieu, en tant qu'intermédiaire.

Le procédé selon l'invention permet d'obtenir les N-carboxyanhydrides de la plupart des α-amino-acides et de leurs dérivés, cycliques ou non, naturels ou synthétiques, dont la fonction amine est primaire ou secondaire et notamment de tous ceux déjà connus pour réagir avec le phosgène, le diphosgène et/ou le triphosgène.

De même, il est très utile pour obtenir les N-carboxyanhydrides des β- et γ-amino-acides et de leurs dérivés, à fonction amine primaire ou secondaire. Ces composés sont en effet considérés comme difficiles à préparer selon les procédés antérieurs.

Les amino-acides qui sont utilisés comme composés de départ sont de préférence les α-, β- ou γ-amino-acides dont le ou les carbones α, β et γ le cas échéant, situés entre le groupe acide et le groupe amino réactifs, forment une chaîne hydrocarbonée alkyle, substituée ou non, pouvant être comprise en totalité ou en partie dans un radical alkyle linéaire ou ramifié, substitué ou non, et/ou dans un cycle alkyle ou hétéroalkyle, substitué ou non. Les substituants sont les groupes ou atomes que l'on trouve habituellement dans les amino-acides, tels que, par exemple, les groupes hydroxy, carboxy, mercapto, alkylthio, alkyldithio, alkyle, cycloalkyle, hétérocycloalkyle, aryle, hétéroaryle, alkyloxy, aryloxy, les atomes d'halogènes, tels que de fluor, de chlore, de brome ou d'iode, les groupes amino, guanidino ou amido substitués ou non par des groupes alkyles.

Plus précisément dans les amino-acides considérés les groupes alkyles comportent de 1 à 7 atomes de carbone et sont substitués ou non par les substituants précédemment indiqués. Les groupes aryles sont non substitués ou substitués par des substituants choisis parmi les atomes d'halogène tels que de fluor, de chlore, de brome ou d'iode et les groupes alkyle, alkoxy, aryloxy, aryle, mercapto, alkylthio, hydroxy, carboxy, amino, alkylamino, dialkylamino, nitro, trifluorométhyle. Lorsqu'ils sont présents, ces groupes substituants sont plus particulièrement en nombre de un à trois. Les groupes aryles sont en particulier les radicaux phényle ou naphtyle substitués ou non.

Les groupes cycloalkyles sont constitués par des cycles ayant de 3 à 7 atomes de carbone, subtitués ou non. Les hétérocycles, qui peuvent être substitués ou non, sont des groupes cycloalkyles ou aryles qui comportent dans le cycle au moins un hétéroatome choisi parmi l'atome d'azote, d'oxygène ou de soufre.

Les substituants des groupes cycloalkyles ou hétérocycloalkyles sont choisis parmi les substituants précédemment indiqués pour les radicaux alkyles et aryles. Les substituants des groupes hétéroaryles sont choisis parmi les substituants indiqués pour les groupes aryles.

Les groupes hétéroaryles sont de préférence des groupes 2- ou 3-furanyle, 2- ou 3-thiényle, 2-, 3- ou 4-pyridinyle, 4-imidazolyle et 3-indolyle, substitués ou non.

Les amino-acides peuvent être sous leurs différentes formes et notamment lorsqu'ils possèdent un ou plusieurs carbones asymétriques sous leurs différentes formes énantiomériques, mélanges soit racémiques ou de diastéréoisomères ou encore sous forme de stéréoisomères purs.

Lorsque le radical de l'amino-acide contient des groupes fonctionnels, autres que le groupe amino et le groupe acide formant le cycle anhydride, susceptibles de réagir dans les conditions du procédé, on les masque par des groupes protecteurs, de façon connue.

Comme exemples d'amino-acides, on peut citer les amino-acides les plus courants tels que la glycine, alanine, valine, leucine, isoleucine, phénylalanine, sérine, thréonine, lysine, δ-hydroxylysine, arginine, ornithine, acide aspartique, asparagine, acide glutamique, glutamine, cystéine, cystine, méthionine, tyrosine, thyroxine, proline, hydroxyproline, tryptophane, l'histidine et leurs dérivés.

Le groupe amino réactif peut être un groupe amino primaire ou secondaire. Par conséquent, l'atome d'azote peut porter un radical aliphatique, cycloaliphalique, araliphatique ou aryle, substitué ou non, comme il est habituel pour la classe des amines. En particulier, ce radical peut être substitué par les groupes précédemment indiqués comme substituants.

Le radical du groupe amino peut également former un cycle, non substitué ou substitué comme précédemment indiqué, avec le reste du radical de l'amino-acide, tel que par exemple dans la proline.

Lorsque le radical comporte des groupes réactifs, on les protège de façon classique.

Comme radical de ce groupe amino, on peut en particulier citer les groupes alkyles, cycloalkyles ou aralkyles non substitués ou substitués, par exemple par des groupes tels que décrits dans le brevet US n°4, 686, 295 pour les nouveaux NCA formés au moyen du phosgène, et en particulier substitués par un ou plusieurs groupes choisis parmi les groupes alkoxycarbonyles, aryloxycarbonyles et aralkyloxycarbonyles.

A la place de l'amino-acide, on peut utiliser un de ses sels comme composé de départ. Par sels de l'amino-acide, on entend les sels obtenus par réaction du groupe amino avec des acides organiques ou minéraux, tels que par exemple les sulfates, acétates, toluènesulfonates, méthanesulfonates et de préférence les halohydrates, en particulier les chlorhydrates et bromhydrates.

Les chlorhydrates sont les sels préférés.

Le procédé convient bien pour obtenir les N-carboxyanhydrides des amino-acides telles que la N-(1-éthoxycarbonyl-3-phénylpropyl)alanine, la leucine, l'alanine, la N-trifluoracétyl-lysine, l'ester γ-benzylique ou l'ester γ- méthylique de l'acide glutamique.

Pour la mise en oeuvre du procédé, le phosgène, le diphosgène et/ou le triphosgène peuvent être mis à réagir avec l'amino-acide pour former le cycle du N-carboxyanhydride. De préférence, on utilise du phosgène.

Par rapport à l'amino-acide, un grand excès de phosgène n'est pas nécessaire. Ainsi de préférence, on ajoute aux environs de 1 à 2 moles de phosgène par mole d'amino-acide ou de son sel.

Le diphosgène ou le triphosgène sont ajoutés en quantité correspondante afin d'obtenir les mêmes rapports phosgène/amino-acide.

La réaction peut être réalisée dans un solvant aprotique et polaire. Les éthers, notamment le tétrahydrofuranne et le dioxanne peuvent être utilisés mais, de préférence, on choisit un solvant appartenant à la famille des esters aliphatiques.

Des solvants aprotiques et apolaires appartenant à la famille des hydrocarbures aliphatiques et aromatiques chlorés ou non, par exemple le dichlorométhane ou le toluène, peuvent également être utilisés.

Les solvants appartenant à la famille des esters ou des hydrocarbures ont l'avantage de ne pas réagir avec le phosgène ou l'acide chlorhydrique. Leur utilisation est par conséquent plus avantageuse.

Les acétates d'alkyle conviennent bien et en particulier l'acétate d'éthyle.

Selon l'invention, la présence dans le milieu réactionnel d'un composé organique insaturé, possédant au moins une double liaison éthylénique et dont l'un des carbones d'au moins une de ces doubles liaisons éthyléniques est complètement substitué par des substituants différents des atomes d'halogène, est essentielle pour obtenir les NCA avec une pureté améliorée et avec de meilleurs rendements.

Tous les composés qui possèdent au moins une double liaison éthylénique de ce type sur laquelle l'acide chlorhydrique peut s'additionner peuvent être utilisés. Ce composé insaturé ne doit bien sûr pas comporter d'autres groupes et/ou atomes, tels que notamment le groupe nitrile et/ou les atomes d'halogène, pouvant réagir avec les composés présents dans le milieu réactionnel. Il en résulterait de nouvelles impuretés et des baisses de rendement. Si le composé comporte d'autres groupes réactifs, on les protège, de façon connue.

Les composés insaturés ayant une double liaison dont un des carbones est complètement substitué par des groupes hydrocarbyles conviennent bien.

De préférence, on utilise un composé appartenant à la famille des hydrocarbures. Comme exemple de tels composés, on peut citer l'α-pinène et le diisobutène. L'α-pinène est le composé préféré.

La quantité de composé insaturé que l'on utilise est généralement de 1 à 3 moles par mole d'amino-acide ou de 1,5 à 4 moles par mole du sel de l'amino-acide, si ce composé est choisi comme composé de départ, et de préférence respectivement aux environs de 2 moles par mole de l'amino-acide ou aux environs de 3 moles par mole de son sel.

Le composé insaturé peut être présent dans le milieu réactionnel, dès le début de la réaction, mais on peut aussi l'ajouter au cours de celle-ci.

La réaction est généralement effectuée à la température habituelle comprise entre 0°C et 120°C ou égale à ces valeurs, et de préférence comprise entre environ 40°C et environ 90°C.

La pression sous laquelle on opère est généralement la pression atmosphérique. On peut également opérer sous pression réduite notamment jusqu'aux environs de 500 mbar, en particulier aux environs de 700 à 800 mbar.

De préférence, la réaction est effectuée dans des conditions anhydres.

Un des avantages du procédé selon l'invention est que la durée de réaction est raccourcie et peut même être diminuée de moitié par rapport à celle de l'art antérieur, en particulier dans des solvants tels que les esters. Ces derniers solvants étant de plus moins chers, la mise en oeuvre du procédé selon l'invention entraîne une réelle économie.

Lorsque la réaction est terminée, les produits sont isolés selon la procédure classique. Le phosgène et le solvant sont généralement éliminés sous l'effet d'une pression réduite. Les dérivés chlorés obtenus à partir des composés insaturés sont séparés lors de la cristallisation des NCA.

Les rendements obtenus en NCA, après cristallisation, sont nettement améliorés et souvent supérieurs à 90 %. Le taux de chlore hydrolysable est toujours inférieur à 0,05 % et souvent la quantité d'impuretés chlorées est si faible que ce taux ne peut pas être déterminé précisément.

En conséquence, les NCA préparés selon le procédé précédemment décrit pourront être utilisés dans les nombreuses applications pour lesquelles des produits très purs sont exigés et notamment pour l'obtention de produits pharmaceutiques.

Les exemples qui suivent illustrent l'invention sans toutefois la limiter.

### EXEMPLE 1 :

### Préparation du N-carboxyanhydride de la leucine (H-Leu-NCA)

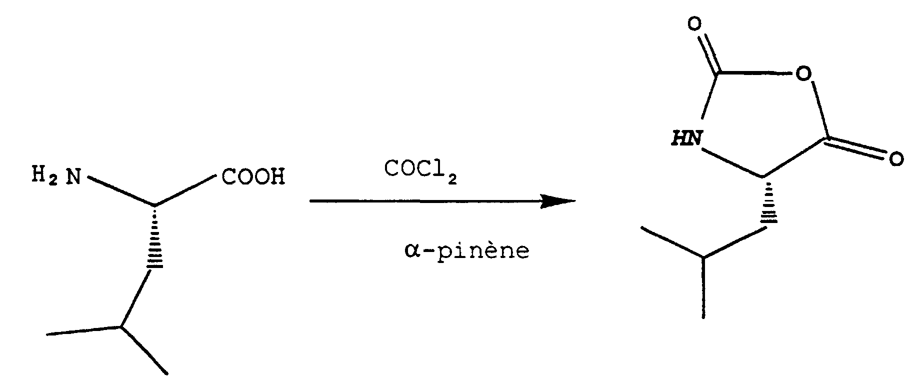

Dans un réacteur thermostaté de 2,5 litres préalablement inerté avec de l'azote, on ajoute 1 litre d'acétate d'éthyle puis 100 g de L-leucine (0,76 mol, 1 équivalent). Dans cette suspension agitée mécaniquement, on introduit 208,0 g d'α-pinène (1,52 mol, 2 équivalents) et on refroidit le mélange à 5°C. On introduit alors, par bullage en une heure, dans ce milieu réactionnel, 154,5 g de phosgène (1,56 mol, 2,05 équivalents) en maintenant la température entre 5°C et 10°C. On chauffe ensuite le milieu réactionnel à 60°-65°C. Au bout de deux heures de palier à cette température, le milieu réactionnel est dégazé sous pression réduite afin d'éliminer le phosgène en excès et afin de le concentrer en éliminant la totalité de l'acétate d'éthyle.

On ajoute ensuite 750 ml d'heptane industriel à chaud sur le milieu concentré. La H-Leu-NCA commence à cristalliser. On refroidit alors le milieu réactionnel à 0°-5°C. On filtre sous atmosphère d'azote. Après séchage sous vide à température ambiante, on obtient 101,9 g (rendement : 85 %) de L-H-Leu-NCA de pureté supérieure à 99,9 % (déterminée par HPLC) et dont le taux de chlore hydrolysable déterminé par la méthode argentimétrique est de 0,018 % en poids.

### EXEMPLE 2 :

### Préparation du N-carboxyanhydride de l'alanine (H-Ala-NCA)

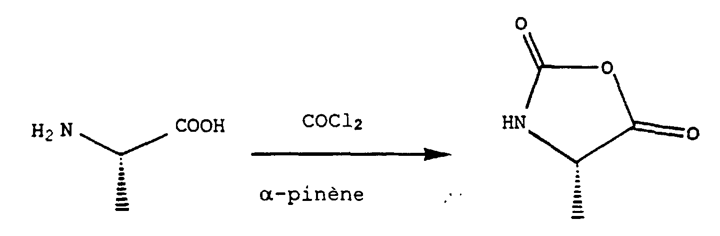

125 g d'alanine (H-Ala-OH) (1,4 mol) sont mis en suspension dans un mélange de 445 ml d'α-pinène (382 g, 2, 8 mol, 2 éq) et 937 ml d'acétate d'éthyle. La suspension est portée à reflux et on introduit 209 g (2,11 mol, 1,5 éq) de phosgène gazeux. Après 12 heures de palier, il reste quelques parties insolubles.

On distille pour séparer, du milieu réactionnel, 800 ml d'un mélange d'acétate d'éthyle et de phosgène puis on filtre le milieu restant à chaud.

On ajoute à chaud, sur le milieu concentré, 800 ml d'heptane industriel et on refroidit à -10°C pendant une nuit. Le produit qui a cristallisé est filtré et lavé à l'heptane industriel.

Après séchage, on obtient 111 g de H-Ala-NCA soit un rendement de 68,8%. La quantité de chlore hydrolysable est trop faible pour être déterminée car elle est inférieure à la limite de détection, c'est-à-dire inférieure à 0,01 %.

### EXEMPLE 3 :

### Préparation du N-carboxyanhydride de la N-trifluoro-acétyl-lysine (H-Lys(TFA)-NCA).

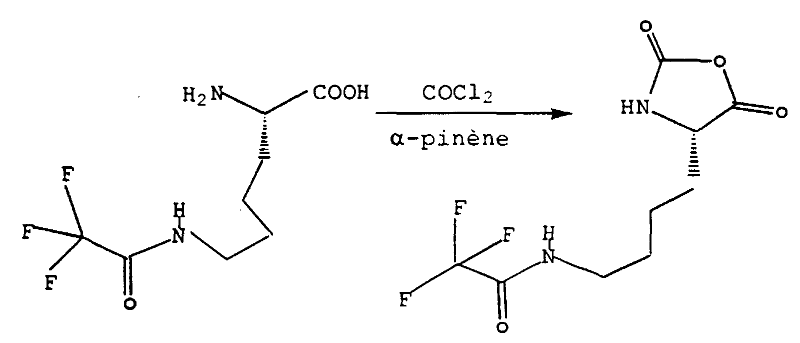

250 g de H-TFA-Lys-OH (1,03 mol) sont mis en suspension dans un mélange de 328 ml d' α-pinène (281 g, 2,06 mol, 2 éq) et 1875 ml d'acétate d'éthyle. On chauffe la suspension à 65°C puis on introduit 154 g (1,55 mol, 1,5 éq) de phosgène gazeux. On porte le milieu réactionnel à reflux et on laisse en palier pendant 3 heures.

On distille pour séparer 1750 ml d'un mélange d'acétate d'éthyle et de phosgène. Dans le milieu restant, on ajoute, à chaud, 1750 ml d'heptane industriel et on refroidit à -10°C pendant une nuit. Le produit qui a cristallisé est séparé par filtration et lavé à l'heptane industriel.

Après séchage, on obtient 261 g de H-Lys(TFA)-NCA soit un rendement de 94,48 %. La quantité de chlore hydrolysable ne peut pas être déterminée car elle est inférieure à la limite de détection, c'est-à-dire inférieure à 0,01 %.

### EXEMPLE 4 :

### Préparation du N-carboxyanhydride de l'ester γ-benzylique de l'acide glutamique (H-Glu(OBzl)-NCA).

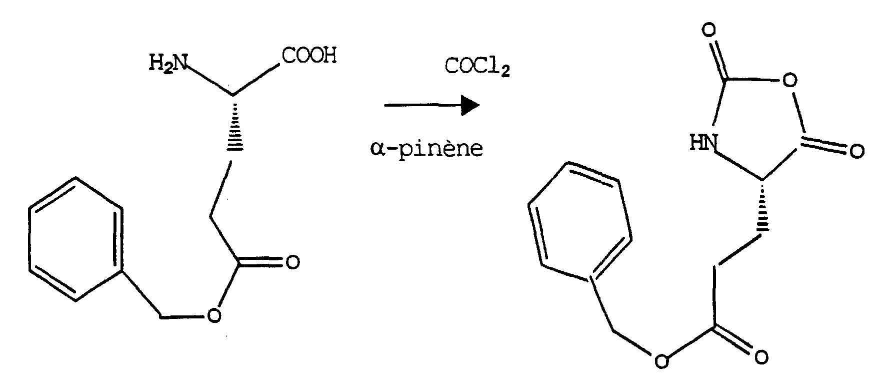

250 g de H-Glu(OBzl)-OH (1,05 mol) sont mis en suspension dans un mélange de 334 ml d' α-pinène (287 g, 2,1 mol, 2 éq) et 1875 ml d'acétate d'éthyle. On refroidit la suspension à + 5°C puis on introduit 164 g (2,28 mol, 1,57 éq) de phosgène gazeux. On chauffe le milieu réactionnel à reflux et on laisse en palier 3 heures à cette température.

On distille ensuite pour séparer 1500 ml d'un mélange d'acétate d'éthyle et de phosgène. On ajoute à chaud, sur le milieu restant 1500 ml d'heptane industriel et on refroidit à -10°C pendant 2 heures. Le produit qui a cristallisé est séparé par filtration et lavé à l'heptane industriel.

Après séchage, on obtient 253 g de H-Glu(OBzl)-NCA, soit un rendement de 91,3 %. Le taux de chlore hydrolysable est indéterminable car il est inférieur à 0,01 % (limite de détection de la méthode).

### EXEMPLE COMPARATIF :

### Préparation du N-carboxyanhydride de l'ester γ-benzylique de l'acide glutamique (H-Glu(OBzl)-NCA)

100 g de H-Glu(OBzl)-OH (0,42 mol) sont mis en suspension dans 885 ml d'acétate d'éthyle. On refroidit la suspension à + 5°C puis on introduit 90 g (0,91 mol, 2,16 éq) de phosgène gazeux.

On porte le milieu réactionnel à reflux. Malgré la présence d'un excès de phosgène supérieur par rapport à l'exemple précédent, la réaction est lente et on est obligé de laisser le milieu réactionnel en palier à la température de reflux, 6 heures au lieu de 3 heures comme dans l'exemple précédent.

On distille ensuite pour séparer 600 ml d'un mélange d'acétate d'éthyle et de phosgène. On ajoute, à chaud, 600 ml d'heptane industriel et on refroidit à - 10°C pendant 2 heures. Le produit cristallisé est séparé par filtration et lavé à l'heptane industriel.

Après séchage, on obtient 88 g de H-Glu(OBzl)-NCA, soit un rendement de 74,6 %. Le taux de chlore hydrolysable est de 0,13 %.

### EXEMPLE 5 :

### Préparation du N-carboxyanhydride de l'ester γ-méthylique de l'acide glutamique (H-Glu(OMe)-NCA).

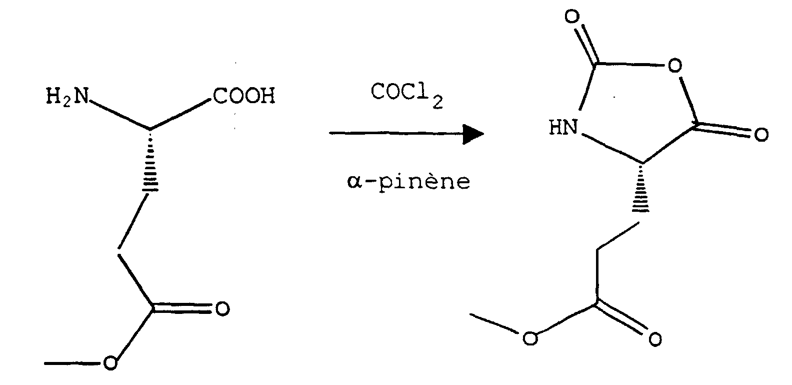

250 g de H-Glu(OMe)-OH (1,55 mol) sont mis en suspension dans un mélange de 493 ml d' α-pinène (423 g, 3,1 mol, 2 éq) et 1875 ml d'acétate d'éthyle. On chauffe la suspension à 65°C puis on introduit 227 g (2,31 mol, 1,5 éq) de phosgène gazeux.

On porte à reflux le milieu réactionnel et on le laisse en palier 6 heures. On distille ensuite pour séparer 1500 ml d'un mélange d'acétate d'éthyle et de phosgène.

On ajoute, à chaud, sur le milieu restant, 1500 ml d'heptane industriel et on refroidit le milieu à -10°C pendant une nuit. Le produit qui a cristallisé est séparé par filtration et lavé à l'heptane industriel.

Après séchage, on obtient 269 g de H-Glu(OMe)-NCA, soit un rendement de 92,6 %. Le taux de chlore hydrolysable est inférieur à 0,01 % (limite de détection).

### EXEMPLE 6 :

### Préparation du N-carboxyanhydride de la N-(1-éthoxycarbonyl-3-phénylpropyl)alanine (EPAL-NCA).

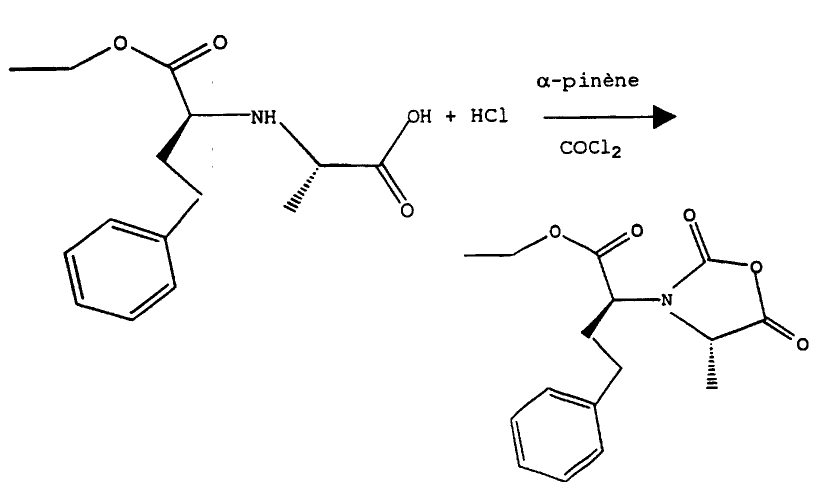

Dans un réacteur thermostaté de 3 litres préalablement inerté à l'azote, on ajoute 2,6 litres d'acétate d'éthyle anhydre puis 312 g d'EPAL (1,11 mol, 1 équivalent). Dans cette suspension agitée mécaniquement, on introduit alors, en 15 minutes à 40°C, 45 g d'HCL gazeux (1,22 mol, 1,1 équivalent/EPAL).

On introduit ensuite dans le milieu réactionnel, en une heure, 223 g de phosgène gazeux (2,22 mol, 2,00 éq). On chauffe ensuite le milieu réactionnel à 60°-65°C. Au bout de deux heures de palier à cette température, on introduit 227 g d'alpha-pinène (1,66 mol, 1,5 éq/EPAL). Après un palier additionnel de 30 minutes, le milieu réactionnel est dégazé sous pression réduite pour éliminer le phosgène en excès et pour séparer la totalité de l'acétate d'éthyle.

On ajoute alors 1385 ml d'éther isopropylique sur le milieu réactionnel concentré. On refroidit le milieu à 0°-5°C et on observe la cristallisation de l'EPAL-NCA. On le sépare par filtration sous atmosphère d'azote.

Après séchage sous vide à température ambiante, on obtient 312 g (rendement : 91,5 %) d'EPAL-NCA (solide blanc) de pureté supérieure à 99,7 % (déterminée par HPLC) et dont le taux de chlore hydrolysable est de 0,04 %.

## Revendications

1. Procédé de préparation des N-carboxyanhydrides par réaction de l'α-, β- ou γ-amino-acide correspondant ou d'un de ses sels, avec le phosgène, le diphosgène et/ou le triphosgène, dans un milieu solvant, **caractérisé en ce que** au moins une partie de la durée de réaction est effectuée en présence d'un composé organique insaturé qui possède une ou plusieurs doubles liaisons éthyléniques, dont le reste de la molécule est inerte vis-à-vis des composés présents dans le milieu, et dont l'un des carbones d'au moins une double liaison éthylénique est complètement substitué par des substituants différents des atomes d'halogène.

2. Procédé selon la revendication 1, **caractérisé en ce que** l'amino-acide est mis à réagir avec le phosgène.

3. Procédé selon la revendication 1 ou 2, **caractérisé en ce que** le composé organique insaturé est choisi parmi les hydrocarbures.

4. Procédé selon la revendication 1 ou 2, **caractérisé en ce que** le composé organique insaturé est l'α-pinène.

5. Procédé selon l'une quelconque des revendications précédentes, **caractérisé en ce que** la quantité du composé insaturé utilisée est de 1 à 3 moles par mole de l'amino-acide ou de 1,5 à 4 moles par mole du sel de l'amino-acide.

6. Procédé selon l'une quelconque des revendications précédentes, **caractérisé en ce que** le solvant est choisi parmi les esters aliphatiques et les hydrocarbures aliphatiques ou aromatiques, chlorés ou non.

7. Procédé selon la revendication 6, **caractérisé en ce que** le solvant est l'acétate d'éthyle.

8. Procédé selon l'une quelconque des revendications précédentes, **caractérisé en ce que** le groupe amino réactif de l'amino-acide est primaire ou secondaire.

9. Procédé selon l'une quelconque des revendications précédentes, **caractérisé en ce que** lorsque l'amino-acide comporte des groupes réactifs autres que le groupe acide et le groupe amino formant l'anhydride, ils sont protégés.

10. Procédé selon l'une quelconque des revendications précédentes, **caractérisé en ce que** l'amino-acide de départ est la leucine, l'alanine, la N-trifluoroacétyl-lysine, l'ester γ-benzylique ou l'ester γ-méthylique de l'acide glutamique, la N-(1-éthoxycarbonyl-3-phénylpropyl)alanine ou un de leurs sels.

11. Procédé selon l'une quelconque des revendications précédentes, **caractérisé en ce que** le sel de l'amino-acide est un sulfate, un acétate, un toluènesulfonate ou un méthanesulfonate.

12. Procédé selon l'une quelconque des revendications précédentes, **caractérisé en ce que** le sel de l'amino-acide est un halohydrate.

## Patentansprüche

1. Verfahren zur Herstellung von N-Carboxyanhydriden durch Umsetzung der entsprechenden α-, β- oder γ-Aminosäure oder einem Salz dieser Aminosäuren mit Phosgen, Diphosgen und/oder Triphosgen in einem Lösungsmittelmedium, **dadurch gekennzeichnet, dass** die Umsetzung zumindest zu einem Teil der Reaktionsdauer in Gegenwart einer ungesättigten organischen Verbindung durchgeführt wird, die eine oder mehrere ethylenische Doppelbindungen besitzt, deren Molekülrest gegenüber den in dem Medium vorliegenden Verbindungen inert ist und bei der eines der Kohlenstoffatome mindestens einer ethylenischen Doppelbindung vollständig mit Substituenten substituiert ist, die von Halogenatomen verschieden sind.

2. Verfahren nach Anspruch 1, **dadurch gekennzeichnet, dass** die Aminosäure mit Phosgen umgesetzt wird.

3. Verfahren nach Anspruch 1 oder 2, **dadurch gekennzeichnet, dass** die ungesättigte organische Verbindung unter den Kohlenwasserstoffen ausgewählt ist.

4. Verfahren nach Anspruch 1 oder 2, **dadurch gekennzeichnet, dass** die ungesättigte organische Verbindung das α-Pinen ist.

5. Verfahren nach einem der vorhergehenden Ansprüche, **dadurch gekennzeichnet, dass** der verwendete Mengenanteil der ungesättigten Verbindung 1 bis 3 mol auf 1 mol Aminosäure oder 1,5 bis 4 mol auf 1 mol Aminosäuresalz beträgt.

6. Verfahren nach einem der vorhergehenden Ansprüche, **dadurch gekennzeichnet, dass** das Lösungsmittel unter den aliphatischen Estern und aliphatischen oder aromatischen Kohlenwasserstoffen, die gegebenenfalls chloriert sind, ausgewählt ist.

7. Verfahren nach Anspruch 6, **dadurch gekennzeichnet, dass** es sich bei dem Lösungsmittel um das Ethylacetat handelt.

8. Verfahren nach einem der vorhergehenden Ansprüche, **dadurch gekennzeichnet, dass** die reaktive Aminogruppe der Aminosäure eine primäre oder eine sekundäre Aminogruppe ist.

9. Verfahren nach einem der vorhergehenden Ansprüche, **dadurch gekennzeichnet, dass**, falls die Aminosäure neben der Säuregruppe und der Aminogruppe, die das Anhydrid bilden, weitere reaktive Gruppen enthält, diese geschützt werden.

10. Verfahren nach einem der vorhergehenden Ansprüche, **dadurch gekennzeichnet, dass** es sich bei der anfänglich eingesetzten Aminosäure um Leucin, Alanin, N-Trifluoracetyllysin, den γ-Benzylester oder γ-Methylester der Glutaminsäure, das N-(1-Ethoxycarbonyl-3-phenylpropyl)alanin oder eines ihrer Salze handelt.

11. Verfahren nach einem der vorhergehenden Ansprüche, **dadurch gekennzeichnet, dass** es sich bei dem Salz der Aminosäure um ein Sulfat, Acetat, Toluolsulfonat oder Methansulfonat handelt.

12. Verfahren nach einem der vorhergehenden Ansprüche, **dadurch gekennzeichnet, dass** das Salz der Aminosäure ein Hydrohalogenid ist.

## Claims

1. A process for the preparation of N-carboxyanhydrides by reacting the corresponding α-, β- or γ-amino acid or one of the salts thereof with phosgene, diphosgene and/or triphosgene in a solvent medium, **characterised in that** at least part of the duration of the reaction proceeds in the presence of an unsaturated organic compound which has one or more ethylenic double bonds, the remainder of the molecule of which is inert towards the compounds present in the medium, and one of the carbons of at least one ethylenic double bond of which is completely substituted by substituents other than halogen atoms.

2. A process according to claim 1, **characterised in that** the amino acid is reacted with phosgene.

3. A process according to claim 1 or 2, **characterised in that** the unsaturated organic compound is selected from among hydrocarbons.

4. A process according to claim 1 or 2, **characterised in that** the unsaturated organic compound is α-pinene.

5. A process according to any one of the preceding claims, **characterised in that** the quantity of unsaturated compound used is from 1 to 3 moles per mole of amino acid or from 1.5 to 4 moles per mole of amino acid salt.

6. A process according to any one of the preceding claims, **characterised in that** the solvent is selected from among aliphatic esters and aliphatic or aromatic hydrocarbons, either chlorinated or non-chlorinated.

7. A process according to claim 6, **characterised in that** the solvent is ethyl acetate.

8. A process according to any one of the preceding claims, **characterised in that** the reactive amino group of the amino acid is primary or secondary.

9. A process according to any one of the preceding claims, **characterised in that** when the amino acid comprises reactive groups other than the acid group and the amino group forming the anhydride, said reactive groups are protected.

10. A process according to any one of the preceding claims, **characterised in that** the starting amino acid is leucine, alanine, N-trifluoroacetyl-lysine, the γ-benzyl ester or γ-methyl ester of glutamic acid, N-(1-ethoxycarbonyl-3-phenylpropyl)alanine or one of the salts thereof.

11. A process according to any one of the preceding claims, **characterised in that** the amino acid salt is a sulfate, an acetate, a toluenesulfonate or a methanesulfonate.

12. A process according to any one of the preceding claims, **characterised in that** the amino acid salt is a hydrohalide.
